# EUROPEAN PATENT APPLICATION

(11) **EP 4 502 132 A1**
(43) Date of publication of application: **05.02.2025**
(21) Application number: 22935286.9
(22) Date of filing: 30.03.2022
(51) Int. Cl.: C12M 1/26, A01N 1/02, A61D 19/02

(54) **CRYOPRESERVATION CONTAINER**

(71) Applicant: Advanced Institute of Reproductive Technologies, Tokyo 160-0022 (JP)
(72) Inventor: KUWAYAMA Masashige, Tokyo 160-0022 (JP); KUWAYAMA Rio, Tokyo 160-0022 (JP)
(74) Representative: Strehl Schübel-Hopf & Partner
(86) International application number: PCT/JP2022/016094
(87) International publication number: WO 2023/188141

(57) **Abstract**

A cryopreservation container 100 includes a cap 3 having a first engagement portion 10 and a body 2 having a second engagement portion 20 at an insertion portion 5. The insertion portion 5 has a biological material loading portion 6, on which a biological material is loaded, at the distal end. The biological material loading portion 6 is housed in a housing 7 defined by the cap 3 and the insertion portion 5 when the insertion portion 5 is inserted through the opening 3b of the cap 3. At this time, the force generated when the first engagement portion 10 and the second engagement portion 20 are engaged with each other enables easy recognition that the cap 3 is successfully joined to the body 2.

## Description

### Technical Field

This disclosure relates to a cryopreservation container.

### Background Art

Various cryopreservation containers have been used to cryopreserve biological materials such as embryos, ova, and spermatozoa of animals. For example, a biological cell cryopreservation tool has been proposed (PTL 1) in which ova are attached together with a small amount of a vitrification solution to a biological cell retaining portion located at an end of the body, and the biological cell retaining portion is immersed in prepared liquid nitrogen to freeze the ova.

This biological cell cryopreservation tool includes a tubular casing that covers the biological cell retaining portion, and the whole tool except the grip at the rear end of the body can be housed in the tubular casing. The body has a tapered portion engageable with the opening of the tubular casing at a portion near the grip. When the tapered portion fits in the opening of the tubular casing, the tubular casing that houses the biological cell retaining portion is sealed.

### Citation List

### Patent Literature

**PTL** 1: International Publication No. 2019/004300

### Summary of Invention

### Technical Problem

It is important for the cryopreservation container that the tubular casing is sealed and contaminants such as germs and foreign substances are prevented from entering the casing in order to cryopreserve biological materials safe without being contaminated. However, it was difficult to check, when the tubular casing is attached to the body, if the opening and the tapered portion fit tightly together without a gap by using the structures of the opening and the tapered portion of the biological cryopreservation tool disclosed in **PTL** 1. The user was required to pull a portion of the biological cell cryopreservation tool out of the liquid nitrogen to check how they fit each time.

Furthermore, the cryopreservation container, which is partly immersed in liquid nitrogen when used, is typically held by tweezers at at least one of the body and the tubular casing. However, for proper fitting of the opening with the tapered portion, the end of the tubular casing of the cryopreservation container needs to be pulled out of the liquid nitrogen temporarily so that the end of the tubular casing is pinched with fingers to fit the opening and the tapered portion tightly together, resulting in poor operating efficiency. Furthermore, it was not clear how much force is needed to push the tapered portion into the opening to properly join the tubular casing to the body. Since different users have different strengths, if the user has a weak pushing force, the user cannot properly join the tubular casing to the body, if the user has a strong pushing force, the cryopreservation container may be damaged. Thus, a cryopreservation container that allows easy recognition that the tubular casing is successfully joined to the body is awaited.

In view of the above, an object of the present disclosure is to provide a cryopreservation container that enables easy recognition that the cap is successfully joined to the body.

### Solution to Problem

The cryopreservation container of the present disclosure includes a cap having a tubular shape and sealed at one end and a body to which the cap is joined, and the container is configured to be used in contact with liquid nitrogen. The cryopreservation container is characterized in that the cap has a first engagement portion on an inner circumferential surface, the body includes an insertion portion having a second engagement portion and a biological material loading portion located at a distal end of the insertion portion and on which a biological material is loaded, and when the insertion portion is inserted through an opening of the cap, the biological material loading portion is housed in a housing defined by the cap and the insertion portion, and a force generated when the first engagement portion and the second engagement portion are engaged with each other enables recognition that the cap is joined to the body.

The cryopreservation container according to the present disclosure is characterized in that each of the first engagement portion integrally formed with the cap and the second engagement portion integrally formed with the insertion portion is formed of a resin composed of polyethylene terephthalate, polypropylene, polyethylene, polycarbonate, cyclic olefin copolymer, polyvinyl chloride, polystyrene, low-density polyethylene, polymethyl methacrylate, polychlorotrifluoroethylene, tetrafluoroethylene hexafluoropropylene copolymer, vinylidene fluoride, or polyamide.

The cryopreservation container according to the present disclosure is characterized in that the first engagement portion includes one or more protrusions.

The cryopreservation container according to the present disclosure is characterized in that the first engagement portion has a sloping surface that slopes toward the opening.

The cryopreservation container according to the present disclosure is characterized in that the cap has a thin-walled portion that is adjacent to the opening and is thinner than a portion adjacent to the one end, and the thin-walled portion has a thickness of 0.05 mm to 0.5 mm.

The cryopreservation container according to the present disclosure is characterized in that the second engagement portion includes an annular protrusion or a plurality of protrusions along an outer periphery of the insertion portion.

The cryopreservation container according to the present disclosure is characterized in that the insertion portion has a larger diameter portion having an outer diameter larger than an inner diameter of the cap, and the housing is sealed by the larger diameter portion fitted in the cap.

The cryopreservation container according to the present disclosure is characterized in that the cap has a smaller diameter portion having an inner diameter smaller than an outer diameter of the insertion portion, and the housing is sealed by the insertion portion fitted in the smaller diameter portion.

The cryopreservation container according to the present disclosure is characterized in that the cap has an outer diameter decreasing from an opening side toward the one end, and the one end of the cap has a stopper having a diameter larger than an outer diameter of the one end.

### Advantageous Effects of Invention

The cryopreservation container according to the present disclosure includes the cap having the first engagement portion and the body having the second engagement portion at its insertion portion. The insertion portion has the biological material loading portion, on which the biological material is loaded, at the distal end. The biological material loading portion is housed in the housing defined by the cap and the insertion portion when the insertion portion is inserted through the opening of the cap. At this time, the first engagement portion and the second engagement portion are engaged with each other, and the force generated when the first and second engagement portions are engaged causes the user to feel a certain resistance and the feeling of the fit caused when the first and second engagement portions are engaged with each other, allowing the user to recognize that the cap is properly joined to the body.

### Brief Description of Drawings

Fig. 1 is a front view of a cryopreservation container according to a first embodiment of the present disclosure with a body and a cap being detached from each other.
Fig. 2 is a front view of the cryopreservation container according to the first embodiment of the present disclosure just before a first engagement portion and a second engagement portion are engaged with each other.
Fig. 3 is a front view of the cryopreservation container according to the first embodiment of the present disclosure with the cap being joined to the body.
Fig. 4 is a magnified view of the inside of the cryopreservation container according to the first embodiment of the present disclosure with the cap being joined to the body.
Fig. 5 is a magnified view of the inside of the cryopreservation container according to the first embodiment of the present disclosure just before the first engagement portion and the second engagement portion are engaged with each other.
Fig. 6 is a magnified view of the inside of the cryopreservation container according to the first embodiment of the present disclosure with the first engagement portion and the second engagement portion being engaged with each other.
Fig. 7 is a plan view of the cap of the cryopreservation container according to the first embodiment of the present disclosure.
Fig. 8 is a magnified view of the inside of a cryopreservation container according to a second embodiment of the present disclosure with a cap being joined to a body and a first engagement portion and a second engagement portion being engaged with each other.
Fig. 9 is a magnified view of the inside of a cryopreservation container according to a third embodiment of the present disclosure with a cap being joined to the body and a first engagement portion and a second engagement portion being engaged with each other.
Fig. 10 is a magnified view of the inside of a cryopreservation container according to a fourth embodiment of the present disclosure with a cap being joined to a body and a first engagement portion and a second engagement portion being engaged with each other.

### Description of Embodiments

### First Embodiment

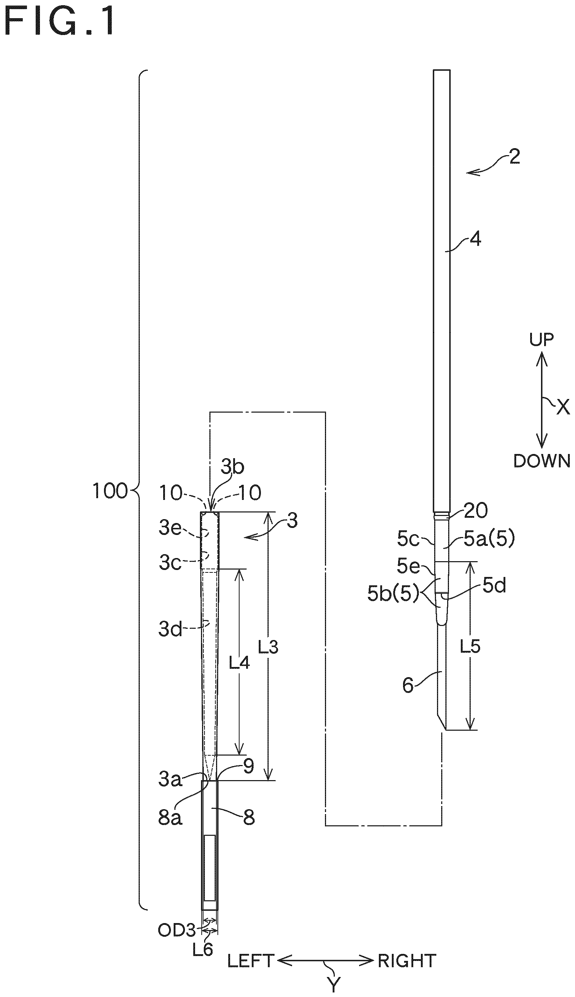

Hereinafter, a first embodiment of the present disclosure will be described with reference to Figs. 1 to 7.

In the embodiments described below, a lengthwise direction of a cryopreservation container 100 illustrated in Fig. 1 is a vertical direction X, a widthwise direction of the cryopreservation container 100 when the cryopreservation container 100 is viewed from the front is a left and right direction Y, and a widthwise direction of the cryopreservation container 100 viewed from the side is a front and rear direction Z. The cryopreservation container 100 is used in liquid nitrogen to cryopreserve biological materials such as embryos, ova, and spermatozoa. In an example of this embodiment, ova are cryopreserved.

As illustrated in Figs. 1 to 3, the cryopreservation container 100 includes a body 2 and a cap 3 joined to the body 2. The body 2 and the cap 3 are formed of a resin that is resistant to liquid nitrogen. The cap 3 is configured to be joined to the body 2 with a portion of the body 2 inserted in the cap 3.

The body 2 has a rod-shaped grip 4 extending downward from the upper end, an insertion portion 5 adjoining the lower end of the grip 4 and tapered in a downward direction, and a plate-shaped biological material loading portion 6 extending downward from the distal end of the insertion portion 5. The grip 4 has a rectangular cuboidal shape in which the lengthwise direction is the vertical direction X, and the user grips the grip 4 when operating the body 2.

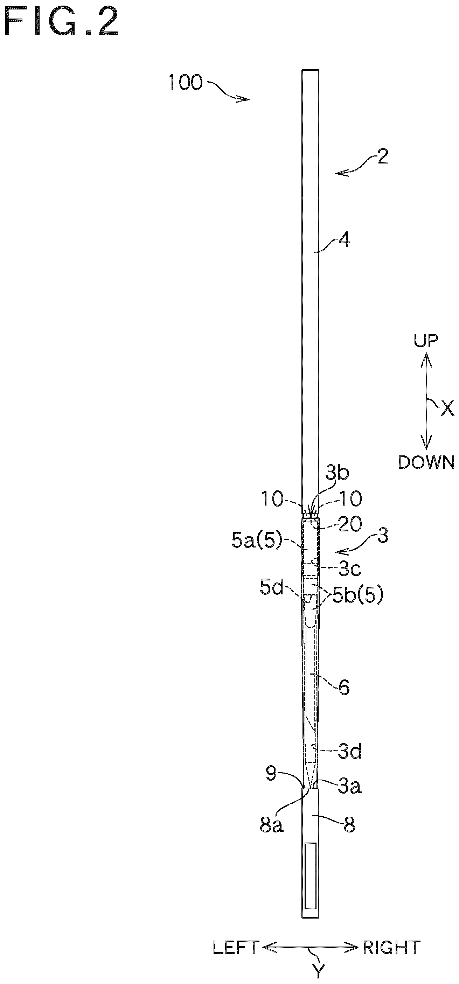

The insertion portion 5 is a portion that is inserted into the cap 3 when the cap 3 is joined to the body 2 (Fig. 2). The insertion portion 5 includes a base 5a adjoining the grip 4 and a holding portion 5b adjoining the lower end of the base 5a. The base 5a has a circular columnar shape in which the lengthwise direction is the vertical direction X, and the holding portion 5b has a conical shape having an outer diameter decreasing in a downward direction from the portion adjoining the base 5a.

The holding portion 5b has a larger diameter portion 5d protruding from and extending along the outer periphery of the holding portion 5b in an annular shape at substantially the middle in the vertical direction X. The holding portion 5 b has the biological material loading portion 6 at the lower end. The biological material loading portion 6 is formed of an elastic sheet or film, and, in the cryopreservation container 100, ova are loaded on the surface of this biological material loading portion 6.

The cap 3, in which the lengthwise direction is the vertical direction X, has a cylindrical shape blocked at a lower end 3a, which is one end. The cap 3 has an opening 3b that opens upward at the upper end, which is the other end. The cap 3 has an outer diameter gradually decreasing from the opening 3b side toward the lower end 3a. The thickness of the resin forming the cap 3 is smaller at a portion adjacent to the opening 3b than at a portion adjacent to the lower end 3a, and this portion having a smaller thickness is a thin-walled portion 3c. The thickness of the thin-walled portion 3c is preferably from 0.05 mm to 0.5 mm. Furthermore, since the resin hardens more readily at cryogenic temperature than at room temperature in liquid nitrogen, the thickness is more preferably 0.05 mm to 0.3 mm to allow smooth joining of the cap 3. In this embodiment, the thickness of the thin-walled portion 3c is 0.15 mm.

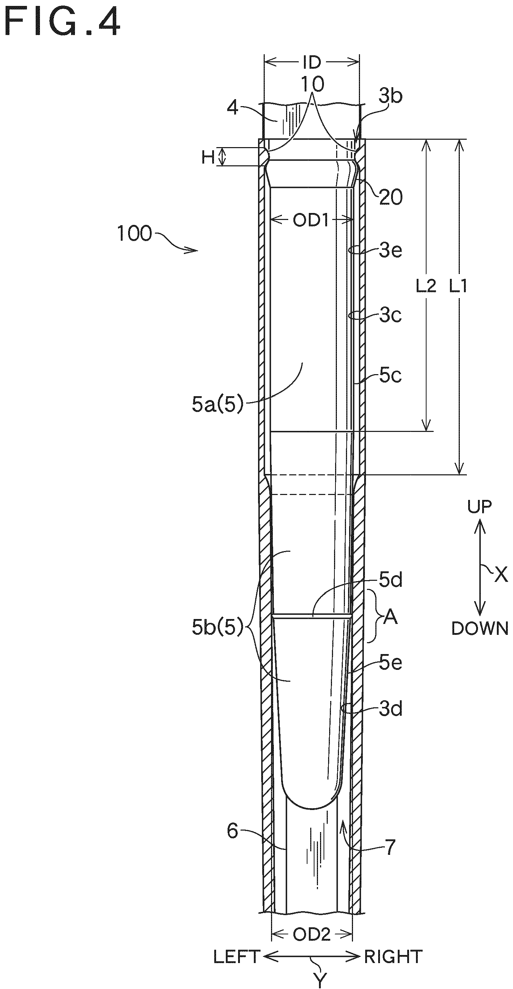

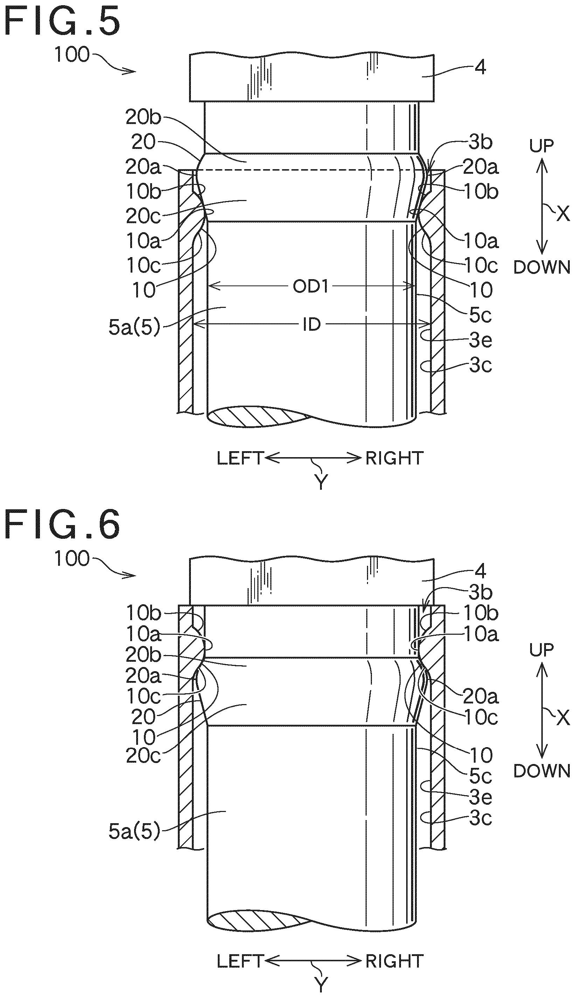

Furthermore, as illustrated in Figs. 4 and 5, the length L1 of the thin-walled portion 3c in the vertical direction X is longer than the length L2 of the base 5a, and the inner diameter ID is larger than the outer diameter OD1 of the base 5a. The length L1 is 0.9 mm, which is about a quarter of the length L3 from the opening 3b to the lower end 3a of the cap 3.

The cap 3 has a smaller diameter portion 3d adjoining the lower end of the thin-walled portion 3c. The smaller diameter portion 3d is a portion in which the resin forming the cap 3 is thicker than the thin-walled portion 3c and extends toward the lower end 3a of the cap 3. The length L4 of the smaller diameter portion 3d in the vertical direction X is longer than the length L5 from the upper end of the holding portion 5b to the lower end of the biological material loading portion 6 (Fig. 1), so that the holding portion 5b and the biological material loading portion 6 can be housed in the smaller diameter portion 3d.

The smaller diameter portion 3d has an inner diameter gradually decreasing toward the lower end 3a. The inner diameter of the smaller diameter portion 3d is slightly larger than the outer diameter OD2 of the larger diameter portion 5d at a portion near the thin-walled portion 3c and is smaller than the outer diameter OD2 of the larger diameter portion 5d at the other portions (Fig. 4). Thus, when the larger diameter portion 5d is inserted in the tapered smaller diameter portion 3d, the larger diameter portion 5d fits in the fitting area A of the smaller diameter portion 3d, which has an inner diameter equal to the outer diameter OD2 of the larger diameter portion 5d. Then, the larger diameter portion 5d that is pushed in the fitting area A seals a housing 7.

As described above, the insertion portion 5 and the biological material loading portion 6 are inserted in the cap 3. When the cap 3 is joined to the body 2, the cap 3 and the holding portion 5b define the housing 7, which is a sealed space, in the cap 3 (Fig. 4). In the cryopreservation container 100, the biological material loading portion 6 is housed in this housing 7.

As illustrated in Figs. 5 and 6, the cap 3 has first engagement portions 10. The first engagement portions 10 are protrusions on an inner circumferential surface 3e of the thin-walled portion 3c and are adjacent to the opening 3b. The first engagement portions 10 are integrally formed with the cap 3 and are opposed to each other. The first engagement portions 10 engage with a second engagement portion 20 when the cap 3 is joined to the body 2.

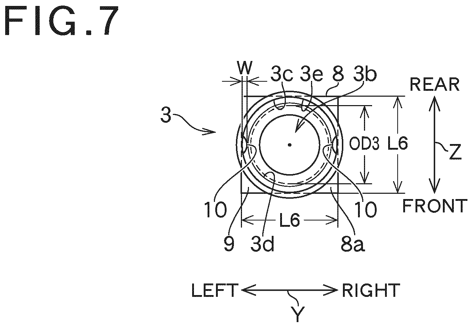

As illustrated in Fig. 7, the first engagement portions 10 are curved protrusions each raised from the inner circumferential surface 3e. The first engagement portion 10 has an upper side surface 10b, which is a side surface adjacent to the upper end or the opening 3b, and a lower side surface 10c, which is a side surface adjacent to the lower side, with a top portion 10a, which is a top, therebetween. The upper side surface 10b and the lower side surface 10c are sloping surfaces that slope upward or downward from the top portion 10a while curving (Figs. 5 and 6).

The first engagement portion 10 has a width W of 0.13 mm in the radial direction of the cap 3. This width W is preferably from 0.01 mm to 0.8 mm.

The first engagement portion 10 has a length in the vertical direction X, i.e., the height H of 0.6 mm (Fig. 4). This height H is preferably from 0.45 mm to 0.85 mm. The width W and the height H of the first engagement portion 10 can be arbitrarily determined by taking the clearance between the inner periphery of the thin-walled portion 3c and the outer periphery of the base 5a into consideration.

The second engagement portion 20 extends along the outer periphery of the base 5a of the insertion portion 5 in an annular shape and is a protrusion protruding from the outer circumferential surface 5c of the base 5a (Fig. 5). The second engagement portion 20 has an upper side surface 20b, which is a side surface adjacent to the upper end, and a lower side surface 20c, which is a side surface adjacent to the lower end, with a top portion 20a, which is a top end of the second engagement portion 20, therebetween.

The lower side surface 20c of the second engagement portion 20 slopes more gently than the upper side surface 20b. Thus, when the cap 3 is joined to the body 2, the first engagement portion 10 is pressed by the lower side surface 20c and thus the thin-walled portion 3c is gradually pushed and widened. Then, the body 2 is pushed further into the cap 3, and the first engagement portion 10, which was moved beyond the top portion 20a of the second engagement portion 20, engages with the upper side surface 20b (Fig. 6).

In the cryopreservation container 100, the second engagement portion 20 protruding from the outer periphery of the base 5a has the same width but may have multiple protrusions arranged in an annular shape to form a corrugated pattern.

The larger diameter portion 5d extends along the outer periphery of the holding portion 5b in an annular shape at substantially the middle of the holding portion 5b in the vertical direction X (Fig. 4). The larger diameter portion 5d protrudes from the outer circumferential surface 5e of the holding portion 5b and has an outer diameter OD2 that is larger than the inner diameter of the smaller diameter portion 3d except for the portion near the thin-walled portion 3c.

In an example of this embodiment, the insertion portion 5 includes the larger diameter portion 5d, the base 5a, the holding portion 5b, and the second engagement portion 20, which are integrally formed. However, the larger diameter portion 5d, the base 5a, the holding portion 5b, and the second engagement portion 20 may be separately formed components.

A weight 8 is a rectangular cuboidal portion at the lower end 3a of the cap 3 (Fig. 1). The rectangular cuboidal weight 8 in which the lengthwise direction is the vertical direction X has an upper surface 8a adjoining the lower end 3a of the cap 3 and is integrally formed with the cap 3.

The shape of the weight 8 is not limited to the rectangular cuboidal shape and may be a circular columnar shape or a polygonal columnar shape.

The upper surface 8a of the weight 8 and the lower end 3a of the cap 3 form a stopper 9. Specifically, the upper surface 8a of the weight 8, which has a rectangular shape, has sides having a length L6 longer than the outer diameter OD3 of the lower end 3a of the cap 3 (Fig. 7). Thus, the upper surface 8a has portions sticking out from the portion adjoining the lower end 3a in the direction around the axis of the cap 3. The portion sticking out in the direction around the axis is the stopper 9.

When the upper surface 8a is circular, the diameter of the upper surface 8a is made larger than the outer diameter OD3 of the lower end 3a, and when the upper surface 8a is polygonal, the diagonal of the upper surface 8a is made longer than the outer diameter OD3 of the lower end 3a to form the stopper 9.

The body 2 and the cap 3, which comes in contact with liquid nitrogen during use, are formed of a resin that is resistant to the temperature of liquid nitrogen. Examples of the resin used for the body 2 and the cap 3 include resins such as polyethylene terephthalate (PET), polypropylene (PP), polyethylene (PE), polycarbonate (PC), cyclic olefin copolymer (COC), and polyurethane (PU).

In addition to the above resins, further examples of the resin include vinyl resins such as polyvinyl chloride (PVC), polyvinylidene chloride (PVDC), and polyvinyl alcohol (PVA), polystyrene resins such as polystyrene (PS), high-density polyethylene (HDPE), medium-density polyethylene (MDPE), low-density polyethylene (LDPE), and ethylene-vinyl acetate copolymer (EVA), acrylic resins such as polymethyl methacrylate (PMMA) and methacrylate-styrene copolymer (MS), fluorinated resins such as polychlorotrifluoroethylene (PCTFE), polytetrafluoroethylene (PTFE), tetrafluoroethylene hexafluoropropylene copolymer (FEP), and polyvinylidene fluoride (PVDF), polyamide resins such as polyamide (PA), polyacetal (POM), and cellulose acetate (CA).

Polyethylene terephthalate and polystyrene are particularly preferred because of their high transparency, which allows the ova on the biological material loading portion 6 to be readily viewed during use in liquid nitrogen or observation of the ova under a microscope. Furthermore, polyethylene terephthalate is preferably used for the biological material loading portion 6 because of its high resistance to solvents such as ethylene glycol, propylene glycol, dimethyl sulfoxide, and glycerin, which are used as cryoprotectants for ova.

Next, how to use the cryopreservation container 100 will be described.

First, ova are attached to the surface of the distal end portion of the biological material loading portion 6 together with a vitrification solution containing a cryoprotectant. Then, at least the biological material loading portion 6 of the body 2 to which the ova are attached are immersed in liquid nitrogen, which has been prepared in advance in the container, to freeze (vitrify) the ova. Then, the cap 3 held with tweezers is immersed in the liquid nitrogen, and the biological material loading portion 6 and the insertion portion 5 are inserted into the cap 3 after the temperature is sufficiently lowered.

Here, when the insertion portion 5 is inserted to the position illustrated in Fig. 2, the second engagement portion 20 of the base 5a comes in contact with the first engagement portion 10 of the cap 3, which causes the user to feel resistance. The cryopreservation container 100 is then stood upright so that the body 2 is on the upper side and the cap 3 is on the lower side, and the body 2 is pushed further into the cap 3 with the lower end of the weight 8 being in contact with the bottom of the container. Then, the second engagement portion 20 moves beyond the top portion 10a of the first engagement portion 10 and engages with the first engagement portion 10 (Fig. 6).

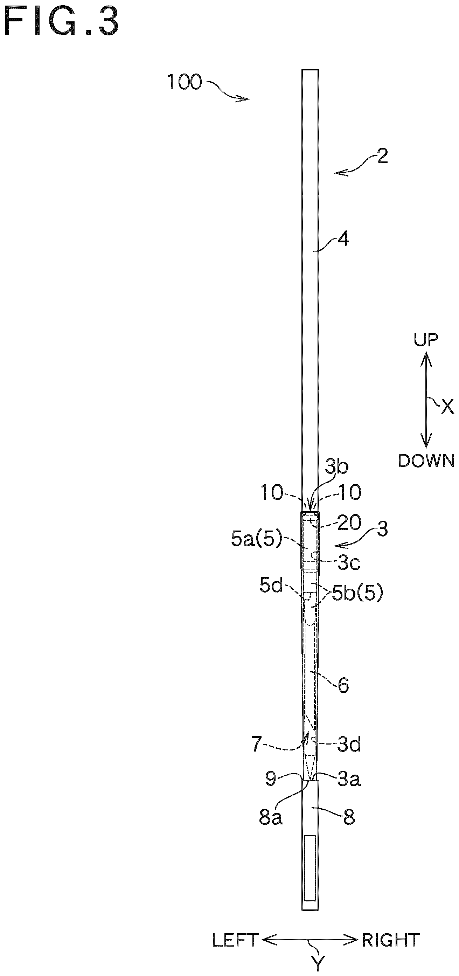

This causes the user's fingers to feel the feeling of the fit caused when the first and second engagement portions 10 and 20 are engaged with each other and also allows the cap 3 to be joined to the body 2 (Fig. 3). The ova and the biological material loading portion 6 are housed in the housing 7 formed by the cap 3 and the insertion portion 5. The housing 7 is sealed when the larger diameter portion 5d is pushed and fitted in the smaller diameter portion 3d of the cap 3 (Fig. 4).

Then, multiple cryopreservation containers 100 each housing ova in the same way are put together in a goblet, and then multiple goblets are put together to be immersed in liquid nitrogen in a liquid nitrogen tank for storage.

Next, the advantages of the cryopreservation container 100 will be described.

In the cryopreservation container 100, when the user joins the cap 3 to the body 2, the force generated when the first and second engagement portions 10 and 20 are engaged with each other causes the user to gain the feeling of the fit. This feeling of the fit allows the user to readily recognize that the cap 3 is successfully joined to the body 2.

The user usually grasps the grip 4 with the fingers of one hand to hold the body 2 while keeping the cap 3 in liquid nitrogen by using tweezers with the other hand to proceed with the cryopreservation process described above. At this time, in the cryopreservation container 100, the cap 3 can be readily joined to the body 2 by simply pushing the body 2 lightly toward the cap 3 using the bottom of the container while supporting the cap 3 with the tweezers. Furthermore, in that state, it is readily recognizable that the cap 3 is successfully joined to the body 2, eliminating the need for pulling the cap 3 out of the liquid nitrogen to check how they are joined together and allowing efficient operation.

In conventional cryopreservation containers, in order to successfully join the cap to the body, it was necessary to pull the end of the cap held with tweezers once out of the liquid nitrogen and pinch the end of the cap, which had been cooled and hardened by the liquid nitrogen, with fingers to firmly join the cap to the body. In addition, when the cap is pinched with fingers, the temperature of the fingers may be transferred to the liquid nitrogen in the cap. This may heat and vaporize a portion of the liquid nitrogen, and the volume expansion caused by vaporization may cause the cap to come off.

The cryopreservation container 100 allows the user to adjust the force to push the body 2 using the feeling of the fit as a guide, and thus the user will not push with greater force than necessary, preventing deformation of the cryopreservation container 100.

Furthermore, the cryopreservation container 100 does not require the end of the cap 3 (the other end having the opening 3b) to be pulled out of the liquid nitrogen, enabling a series of operations to join the cap 3 to the body 2 to be performed in the liquid nitrogen. Thus, the cap 3 can be joined to the body 2 without the operator's fingers touching the liquid nitrogen at the end of the cap 3. Furthermore, since the operator can join the cap 3 to the body 2 without touching the end of the cap 3, the liquid nitrogen in the cap 3 does not vaporize, and the cap 3 can be reliably joined to the body 2.

In the cryopreservation container 100, the first engagement portion 10 is integrally formed with the cap 3, and the second engagement portion 20 is integrally formed with the insertion portion 5. Each of the first engagement portion 10 and the second engagement portion 20 is formed of, among the above-described resins, a resin composed of polyethylene terephthalate, polypropylene, polyethylene, polycarbonate, cyclic olefin copolymer, polyvinyl chloride, polystyrene, low-density polyethylene, polymethyl methacrylate, polychlorotrifluoroethylene, tetrafluoroethylene hexafluoropropylene copolymer, vinylidene fluoride, or polyamide. Thus, the first and second engagement portions 10 and 20 have a certain elasticity even in the cryogenic environment in liquid nitrogen, in contrast with those made of other materials such as metal. This enables the user, even in a cryogenic environment, to gain the feeling of the fit caused when the first and second engagement portions 10 and 20 are engaged with each other and to reliably join the cap 3 to the body 2.

In the cryopreservation container 100 in which the first engagement portion 10 includes two protrusions, the cap 3 can be joined to the body 2 more smoothly than in a configuration in which the first engagement portion is a recess.

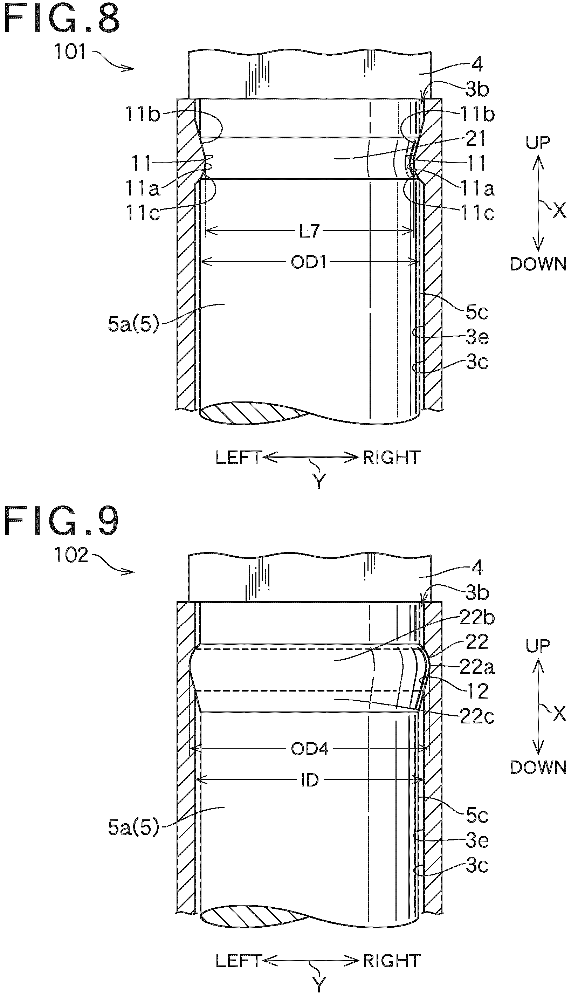

As the cryopreservation container 102 according to the third embodiment, which will be described below (Fig. 9), the first engagement portion 12 may be a protrusion. In such a case, the second engagement portion 22 protruding from the outer circumferential surface 5c of the base 5a in the direction around the axis has the maximum outer diameter OD4 larger than the inner diameter **ID** of the thin-walled portion 3c of the cap 3. Thus, when the cap 3 is joined to the body 2, the second engagement portion 22 is pushed into the cap 3 while pressing the inner circumferential surface 3e of the thin-walled portion 3c after contact with the edge of the opening 3b. This always creates resistance because the inner circumferential surface 3e of the thin-walled portion 3c is always be pressed by the second engagement portion 22 until the second engagement portion 22 reaches the position of the first engagement portion 12.

In contrast, when the first engagement portion 10 is a protrusion, the maximum outer diameter of the second engagement portion 20 including the top portion 20a can be made smaller than the inner diameter **ID** of the thin-walled portion 3c of the cap 3, which allows a clearance to be provided between the top portion 20a of the second engagement portion 20 and the thin-walled portion 3c of the cap 3. Thus, the cap 3 can be joined smoothly without resistance.

Furthermore, when the first engagement portion 10 is a protrusion, it is also easy to form the first engagement portion 10 that provides the desired feeling of the fit by taking various conditions such as the clearance between the inner periphery of the cap 3 and the outer periphery of the insertion portion 5 and the shape and size of the second engagement portion 20 into consideration.

In the cryopreservation container 100, the first engagement portion 10 has the upper side surface 10b, which is a sloping surface sloping toward the opening 3b. Thus, when the cap 3 is joined to the body 2, the cap 3 is pushed with the second engagement portion 20 being in contact with the upper side surface 10b, and the second engagement portion 20 smoothly moves beyond the top portion 10a of the first engagement portion 10. Thus, the user can join the cap 3 to the body 2 with appropriate force without creating more resistance than necessary, thus ensuring safe use.

In the cryopreservation container 100, the cap 3 has the thin-walled portion 3c adjacent to the opening 3b, and the thickness of this thin-walled portion 3c is 0.15 mm. This enables the thin-walled portion 3c to have a certain elasticity even in a cryogenic environment in liquid nitrogen, allowing the cap 3 to be smoothly joined to the body 2.

The second engagement portion 20 is a protrusion extending in an annular shape along the outer periphery of the base 5a of the insertion portion 5. This enables, when the cap 3 is joined to the body 2, the first engagement portion 10 to successfully engage with the second engagement portion 20 regardless of the relative positions between the first engagement portion 10 and the second engagement portion and 20. This eliminates the need for alignment between the first engagement portion 10 and the second engagement portion 20, resulting in an improvement in operation efficiency.

The insertion portion 5 has the larger diameter portion 5d having the outer diameter OD2 larger than the inner diameter of the smaller diameter portion 3d. In other words, the cap 3 has the smaller diameter portion 3d having an inner diameter smaller than the outer diameter OD2 of the larger diameter portion 5d. When the larger diameter portion 5d of the insertion portion 5 is fitted in the smaller diameter portion 3d in the fitting area A, the housing 7 is sealed. Thus, the cap 3 joined to the body 2 prevents contaminants outside the cryopreservation container 100 from entering the housing 7, enabling safe cryopreservation of ova.

The cap 3 decreases in outer diameter from the opening 3b side toward the lower end 3a and has the stopper 9 at the lower end 3a that has a diameter larger than the outer diameter OD3 of the lower end 3a. Thus, for detachment of the cap 3 from the body 2, the user holds the cap 3 with tweezers and slides the tweezers from the opening 3b side toward the lower end 3a, allowing the tweezers to be caught on the stopper 9. This enables the cap 3 to be readily detached from the body 2 by using the stopper 9.

This eliminates the need for the cap 3 to be pulled out of the liquid nitrogen and to be detached with fingers, resulting in an improvement in operation efficiency. Furthermore, since the cap 3 can be detached in the cryogenic environment in liquid nitrogen, the ova are less likely to be influenced by rapid temperature changes.

The cryopreservation container 100 further includes the weight 8. When the cap 3 is immersed in liquid nitrogen, the portion having the weight 8 can be readily turned downward, and thus it is easier to keep the cap 3 and the cryopreservation container 100 upright. This allows the user to readily join or detach the cap 3 even when the cap 3 or a portion of the cryopreservation container 100 is immersed in liquid nitrogen.

### Second Embodiment

Next, a second embodiment of this disclosure will be described with reference to Fig. 8.

A cryopreservation container 101 of this embodiment differs from the cryopreservation container 100 of the first embodiment mainly in the first and second engagement portions. Thus, first and second engagement portions 11 and 21 of the cryopreservation container 101 will be mainly described below. The same reference numerals are assigned to the same components as those in the cryopreservation container 100 without explanation.

In the cryopreservation container 101, the outer diameter OD1 of the base 5a of the insertion portion 5 is larger than that in the cryopreservation container 100, and the base 5a has a recessed second engagement portion 21. Specifically, the second engagement portion 21 is formed by recessing the outer circumferential surface 5c of the base 5a in an annular shape along the outer periphery of the base 5a.

Furthermore, in the cryopreservation container 101, the inner circumferential surface 3e of the thin-walled portion 3c has two first engagement portions 11 opposed to each other. The first engagement portion 11 is a protrusion that can engage with the recess of the second engagement portion 21. Furthermore, a length L7 between the top portions 11a, which is the tops of the two first engagement portions 11, is shorter than the outer diameter OD1 of the base 5a. In contrast with the first engagement portion 10 of the cryopreservation container 100, in the first engagement portion 11, an upper side surface 11b, which is a side surface adjacent to the upper end, is a sloping surface that slopes more gently than a lower side surface 11c, which is a side surface adjacent to the lower end 3a.

In the cryopreservation container 101, due to the combination of the recessed second engagement portion 21 and the protruded first engagement portion 11 described above, when the base 5a is inserted in the thin-walled portion 3c, the base 5a first comes in contact with the first engagement portion 11 and creates resistance. When the base 5a is pushed further downward against this resistance, the first engagement portion 11 is pressed by the base 5a and thus the thin-walled portion 3c is pushed and widened. Then, the first engagement portion 11 reaches the position of the second engagement portion 21 and engages with the second engagement portion 21. At this time, the user gains the feeling of the fit caused when the first engagement portion 11 is engaged with the second engagement portion 21. This enables easy recognition that the cap 3 is successfully joined to the body 2.

### Third Embodiment

Next, a third embodiment of this disclosure will be described with reference to Fig. 9.

A cryopreservation container 102 of this embodiment differs from the cryopreservation container 101 of the second embodiment mainly in the first and second engagement portions. Thus, first and second engagement portions 12 and 22 of the cryopreservation container 102 will be mainly described below. The same reference numerals are assigned to the same components as those in the cryopreservation containers 100 and 101 without explanation.

In the cryopreservation container 102, the inner circumferential surface 3e of the thin-walled portion 3c has the first engagement portion 12 that is a recess. Specifically, the first engagement portion 12 is formed by recessing the inner circumferential surface 3e in an annular shape along the inner periphery of the cap 3.

Furthermore, in the cryopreservation container 102, the second engagement portion 22 is a protrusion that can engage with the recess of the first engagement portion 12. The second engagement portion 22 is an annular protrusion protruding from the outer circumferential surface 5c of the base 5a along the outer periphery of the base 5a. The maximum outer diameter OD4, which includes a top portion 22a, which is the top of the second engagement portion 22, is larger than the inner diameter **ID** of the thin-walled portion 3c. In the second engagement portion 22, a lower side surface 22c, which is a side surface adjacent to the lower end, is a sloping surface that slopes more gently than an upper side surface 22b, which is a side surface adjacent to the upper end.

In the cryopreservation container 102, due to the combination of the recessed first engagement portion 12 and the protruding second engagement portion 22 described above, when the base 5a is inserted in the thin-walled portion 3c, the second engagement portion 22 first comes in contact with the edge of the opening 3b and creates resistance. When the base 5a is pushed further downward against this resistance, the second engagement portion 22 pushes and widens the thin-walled portion 3c. Then, the second engagement portion 22 is further pushed to the position of the first engagement portion 12 and is engaged with the first engagement portion 12. At this time, the user gains the feeling of the fit caused when the second engagement portion 22 is engaged with the first engagement portion 12. This enables easy recognition that the cap 3 is successfully joined to the body 2.

### Fourth Embodiment

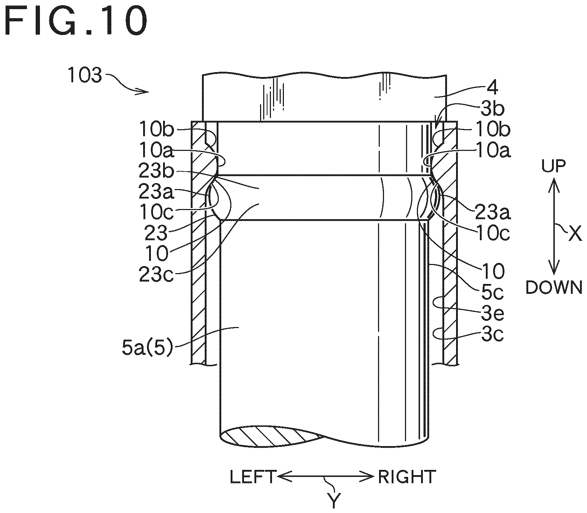

Next, a fourth embodiment of the present disclosure will be described with reference to Fig. 10.

A cryopreservation container 103 of this embodiment differs from the cryopreservation container 100 of the first embodiment mainly in the shape of the second engagement portion. Thus, a second engagement portion 23 of the cryopreservation container 103 will be mainly described below. The same reference numerals are assigned to the same components as those in the cryopreservation container 100 without explanation.

In the cryopreservation container 103, the second engagement portion 23 extends along the outer periphery of the base 5a in an annular shape and is a protrusion protruding from the outer circumferential surface 5c of the base 5a. The second engagement portion 23 has an upper side surface 23b, which is a side surface adjacent to the upper end, and a lower side surface 23c, which is a side surface adjacent to the lower end, with a top portion 23a, which is the top of the second engagement portion 23, therebetween. This lower side surface 23c is a sloping surface that slopes more steeply at a larger angle to the outer circumferential surface 5c of the base 5a than the lower side surface 20c of the second engagement portion 20 of the cryopreservation container 100.

The lower side surface 23c, which is a steep sloping surface, creates greater resistance in the cryopreservation container 103 than the resistance created in the cryopreservation container 100 when the first engagement portion 10 in contact with the lower side surface 23c moves beyond the top portion 23a of the second engagement portion 23. The user will then feel a greater resistance when the second engagement portion 23 and the first engagement portion 10 are engaged with each other and can gain a stronger feeling of the fit. Thus, even when the cap 3 is joined while the cryopreservation container 103 being held with tweezers, the user can reliably feel the feeling of the fit and thus can readily recognize that the cap 3 is joined.

Although the embodiments have been described in detail above, the present disclosure should not be limited to the embodiments described above. For example, in the examples of the embodiments, the first engagement portion 10 (11, 12) is integrally formed with the cap 3 and the second engagement portion 20 (21, 22, 23) is integrally formed with the insertion portion 5. However, the first engagement portion 10 (11, 12) may be a separate component from the cap 3, and the second engagement portion 20 (21, 22, 23) may be a separate component from the insertion portion 5. In addition, the first engagement portion 10 (11, 12) and the second engagement portion 20 (21, 22, 23) may be formed of, for example, metal other than resin.

Furthermore, in the examples of the embodiments, the first engagement portions 10 (11) are two protrusions having the same size and opposed to each other. However, for example, one of the first engagement portions 10 (11) may have a different size or shape from the other first engagement portions 10 (11). Furthermore, the number of first engagement portions 10 (11) is not limited to two and may be any number. The number of first engagement portions 10 (11) may be three or more or the number of first engagement portions 10 (11) may be only one. For example, the inner circumferential surface 3e of the thin-walled portion 3c may have four first engagement portions 10 (11) in two pairs. Among them, the opposing first engagement portions 10 (11) may have the same size, and the adjacent first engagement portions 10 (11) may have different sizes.

Furthermore, in the examples of the embodiments, the first engagement portions 10 and 11 have the upper side surfaces 10b and 11b, respectively, which are sloping surfaces sloping toward the opening 3b. However, for example, the first engagement portions 10 and 11 do not have to be the curved protrusions as in the cryopreservation container 100 and may have any shape in which at least the upper side surfaces 10b and 11b adjacent to the opening 3b are sloping surfaces sloping toward the opening. For example, the protrusion may protrude in a trapezoidal or triangular shape. Furthermore, the first engagement portions 10 and 11 may be protrusions having the upper side surfaces 10b and 11b not sloping.

Furthermore, in the examples of the embodiments, the cap 3 has the thin-walled portion 3c adjacent to the opening 3b, but the cap 3 may have no thin-walled portion 3c.

Furthermore, in the example of the embodiments, the cryopreservation container 100 has both the larger diameter portion 5d and the smaller diameter portion 3d. However, the cryopreservation container 100 may have one of the larger diameter portion 5d and the smaller diameter portion 3d to seal the housing 7. For example, the larger diameter portion 5d may be fitted in the cap 3 to seal the housing 7, or the base 5a or the holding portion 5b may be fitted in the smaller diameter portion 3d to seal the housing 7. Furthermore, for example, the second engagement portion 20 may have the function of the larger diameter portion 5d, and the second engagement portion 20 may be fitted in the thin-walled portion 3c or the smaller diameter portion 3d to seal the housing 7.

Furthermore, in the examples of the embodiments, the larger diameter portion 5d is fitted in the fitting area A where the inner diameter of the smaller diameter portion 3d is equal to the outer diameter OD2 of the larger diameter portion 5d. However, the outer diameter OD2 of the larger diameter portion 5d may be larger than the inner diameter of the smaller diameter portion 3d, and the larger diameter portion 5b may be pushed into the smaller diameter portion 3d to seal the housing 7. Furthermore, if there is no need to seal the housing 7, both the larger diameter portion 5d and the smaller diameter portion 3d may be eliminated.

Furthermore, in the examples of the embodiments, the body 2 integrally includes the grip 4, the base 5a, the holding portion 5b, and the biological material loading portion 6. However, the grip 4, the base 5a, the holding portion 5b, and the biological material loading portion 6 may be separate components. For example, the holding portion 5b and the biological material loading portion 6 may be separate components, and the biological material loading portion 6 may be held in a cutout in the distal end of the holding portion 5b.

The shape of the cap 3 is not limited to the cylindrical shape and may be a tubular shape having a polygonal shape in cross section taken in the direction intersecting the longitudinal direction. Furthermore, the insertion portion 5 is also not limited to the circular columnar shape and may be a polygonal columnar shape. Furthermore, the shape of the grip 4 and the weight 8 is also not limited to the rectangular cuboidal shape and may be a circular columnar shape or a polygonal columnar shape. However, it is preferable that the shape of the grip 4 and the weight 8 be a rectangular cuboidal shape or polygonal columnar shape if the cryopreservation containers need to be stacked on top of another or if information about cryopreservation such as start date of cryopreservation need to be written or printed on the grip or the weight.

### Reference Signs List

100, 101, 102, 103 cryopreservation container
2 body
3 cap
3a lower end
3b opening
3c thin-walled portion
3d smaller diameter portion
3e inner circumferential surface
4 grip
5 insertion portion
5a base
5b holding portion
5c outer circumferential surface
5d larger diameter portion
5e outer circumferential surface
6 biological material loading portion
7 housing
8 weight
8a upper surface
9 stopper
10, 11, 12 first engagement portion
10a, 11a top portion
10b, 11b upper side surface
10c, 11c lower side surface
20, 21, 22, 23 second engagement portion
20a, 22a, 23a top portion
20b, 22b, 23b, upper side surface
20c, 22c, 23c lower side surface
A fitting area
H height
ID inner diameter
L1, L2, L3, L4, L5, L6, L7 length
OD1, OD2, OD3 outer diameter
OD4 maximum outer diameter
W width
X vertical direction
Y left and right direction
Z front and rear direction

## Claims

1. A cryopreservation container comprising: a cap having a tubular shape and sealed at one end; and a body to which the cap is joined, the cryopreservation container being configured to be used in contact with liquid nitrogen, wherein
the cap has a first engagement portion on an inner circumferential surface,
the body includes an insertion portion having a second engagement portion and a biological material loading portion located at a distal end of the insertion portion and on which a biological material is loaded, and
when the insertion portion is inserted through an opening of the cap, the biological material loading portion is housed in a housing defined by the cap and the insertion portion, and a force generated when the first engagement portion and the second engagement portion are engaged with each other enables recognition that the cap is joined to the body.

2. The cryopreservation container according to claim 1, wherein each of the first engagement portion integrally formed with the cap and the second engagement portion integrally formed with the insertion portion is formed of a resin composed of polyethylene terephthalate, polypropylene, polyethylene, polycarbonate, cyclic olefin copolymer, polyvinyl chloride, polystyrene, low-density polyethylene, polymethyl methacrylate, polychlorotrifluoroethylene, tetrafluoroethylene hexafluoropropylene copolymer, vinylidene fluoride, or polyamide.

3. The cryopreservation container according to claim 1 or 2, wherein the first engagement portion includes one or more protrusions.

4. The cryopreservation container according to claim 3, wherein the first engagement portion has a sloping surface that slopes toward the opening.

5. The cryopreservation container according to claim 1 or 2, wherein the cap has a thin-walled portion that is adjacent to the opening and is thinner than a portion adjacent to the one end, and the thin-walled portion has a thickness of 0.05 mm to 0.5 mm.

6. The cryopreservation container according to claim 1 or 2, wherein the second engagement portion includes an annular protrusion or a plurality of protrusions along an outer periphery of the insertion portion.

7. The cryopreservation container according to claim 1 or 2, wherein the insertion portion has a larger diameter portion having an outer diameter larger than an inner diameter of the cap, and the housing is sealed by the larger diameter portion fitted in the cap.

8. The cryopreservation container according to claim 1 or 2, wherein the cap has a smaller diameter portion having an inner diameter smaller than an outer diameter of the insertion portion, and the housing is sealed by the insertion portion fitted in the smaller diameter portion.

9. The cryopreservation container according to claim 1 or 2, wherein the cap has an outer diameter decreasing from an opening side toward the one end, and the one end of the cap has a stopper having a diameter larger than an outer diameter of the one end.
